# FASCICULE DE BREVET EUROPEEN

(11) **EP 0 659 741 B1**
(45) Date de publication et mention de la délivrance du brevet: **19.04.2000**
(21) Numéro de dépôt: 94420341.3
(22) Date de dépôt: 06.12.1994
(51) Int. Cl.: C07D 201/08

(54) **Procédé de préparation de lactame**
Verfahren zur Herstellung von Laktamen
Process for the preparation of lactams

(30) Priorité: 23.12.1993 FR 9315775
(43) Date de publication de la demande: 28.06.1995
(73) Titulaire: RHODIA FIBER & RESIN INTERMEDIATES, 92408 Courbevoie Cédex (FR)
(72) Inventeur: Barratt, David, Ramsbottom Bury BL 09LX (GB); Gilbert, Laurent, F-69007 Lyon (FR)
(74) Mandataire: Vignally, Noel

(56) Documents cités:
- EP-A- 0 150 295
- FR-A- 2 029 540
- US-A- 2 357 484
- US-A- 4 628 085
- CHEMICAL ABSTRACTS, vol. 74, no. 18, 3 Mai 1971, Columbus, Ohio, US; abstract no. 91756t, page 271 ;colonne 1 ; & JP-A-7 026 847 (TORAY INDUSTRIES, INC)

## Description

La présente invention concerne la préparation de lactame par hydrolyse cyclisante d'aminonitrile correspondant.

Les lactames aliphatiques, tels que notamment l'epsilon-caprolactame, sont des composés de base pour la préparation des polyamides (polyamide 6 à partir du caprolactame).

Un des moyens connus de préparer ces lactames consiste à effectuer une hydrolyse cyclisante des aminonitriles correspondants, plus particulièrement des aminonitriles aliphatiques non ramifiés, par passage en phase vapeur avec de l'eau sur un catalyseur solide.

Ainsi le brevet US 2 357 484 décrit un procédé de préparation en phase vapeur de lactame, consistant à faire passer un mélange d'eau et d'aminonitrile sur un catalyseur de déshydratation, tel qu'alumine activée, gel de silice et d'oxyde de titane ou acide borophosphorique.

Le brevet US 4 628 085 a proposé un procédé de préparation de lactames en phase vapeur, consistant à mettre en contact un aminonitrile aliphatique ou aromatique et de l'eau avec un catalyseur à base de silice, sous forme de particules sphériques ayant une surface BET supérieure à 250m²/g et un diamètre moyen de pores inférieur à 20 nm, et généralement en présence d'hydrogène et d'ammoniac.

En général, les catalyseurs utilisés dans les procédés de l'art antérieur permettent d'obtenir de bonnes sélectivités en lactame. Par contre, il s'avère souvent que leur désactivation est rapide, ce qui constitue un très gros handicap pour une mise en oeuvre industrielle desdits procédés.

En outre, le procédé selon US 4 628 085 met en oeuvre un mélange réactionnel très complexe, nécessitant en fin de réaction des opérations de séparation et des recyclages qui compliquent beaucoup ledit procédé.

La présente invention propose de nouveaux catalyseurs qui, tout en conduisant à un bonne sélectivité de la réaction de transformation des aminonitriles en lactames, ont une durée de vie plus grande et nécessitent donc une régénération moins fréquente.

Plus précisément, l'invention consiste en un procédé de préparation de lactame par réaction en phase vapeur d'un aminonitrile aliphatique de formule générale (I) :

N≡C-R-NH₂ (I)

dans laquelle R représente un radical alkylène ayant de 3 à 12 atomes de carbone, avec de l'eau, en présence d'un catalyseur solide, caractérisé en ce que le catalyseur est un phosphate métallique de formule générale (II) :

(PO₄)ₙ Hₕ M, (Imp)ₚ (II)

dans laquelle :
- M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O,
- Imp représente un composé d'imprégnation basique constitué d'un métal alcalin ou alcalino-terreux, ou de mélanges de plusieurs de ces métaux, associé à un contre-anion pour assurer la neutralité électrique,
- n représente 1, 2 ou 3,
- h représente 0, 1 ou 2,
- p représente un nombre compris entre 0 et 1/3 et correspond à un rapport molaire entre l'imprégnant Imp et l'imprégné (PO₄)ₙ Hₕ M.

Parmi les aminonitriles de formule (I) les plus importants sont ceux qui conduisent aux lactames servant de matière première pour la préparation des polyamides 4, 5, 6 et 10, c'est-à-dire ceux dans la formule desquels le symbole R représente un radical alkylène linéaire ayant 3, 4, 5 ou 9 atomes de carbone. Le composé de formule (I) préféré est l'amino-6 capronitrile (ou epsilon-capronitrile), qui conduit au caprolactame dont la polymérisation fournit le polyamide 6.

Parmi les métaux des groupes 2a, 3b, 4b ,5b, 6b, 7b, 8, 2b, 3a, 4a et 5a de la classification périodique des éléments, on peut citer notamment le béryllium, le magnésium, le calcium, le strontium, le baryum, l'aluminium, le bore, le gallium, l'indium, l'yttrium, les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium, le zirconium, le titane, le vanadium, le niobium, le fer, le germanium, l'étain, le bismuth.

Parmi les phosphates de lanthanides, on peut distinguer une première famille qui regroupe les orhophosphates de terres rares légères, également dénommées terres rares cériques, incluant le lanthane, le cérium, le praséodyme, le néodyme, le samarium et l'europium. Ces orthophosphates sont dimorphiques. Ils présentent une structure hexagonale et évoluent vers une structure monoclinique, lorsqu'ils sont chauffés à une température de 600 à 800°C.

Une deuxième famille de phosphates de lanthanides regroupe les orthophosphates de gadolinium, de terbium et de dysprosium. Ces orthophosphates présentent la même structure que les orthophosphates de terres rares cériques, mais présentent en plus une troisième phase cristalline de structure quadratique à haute température (vers 1700°C).

Une troisième famille de phosphates de lanthanides regroupe les orthophosphates de terres rares lourdes, appelées également terres rares yttriques, incluant l'yttrium, l'holmium, l'erbium, le thulium, l'ytterbium.et le lutécium. Ces composés cristallisent uniquement sous la forme quadratique.

Parmi les différentes familles d'orthophosphates de terres rares précitées, on fait appel préférentiellement aux orthophosphate de terres rares cériques.

On peut mettre en oeuvre des phosphates métalliques de formule (II) qui sont des mélanges de phosphates de plusieurs des métaux indiqués précédemment ou des phosphates mixtes de plusieurs des métaux indiqués précédemment ou encore des phosphates mixtes contenant un ou plusieurs des métaux indiqués précédemment et un ou plusieurs autres métaux tels que les métaux alcalins ou alcalino-terreux.

Les contre-anions entrant dans la formule du composé d'imprégnation Imp sont basiques. On peut notamment utiliser les ions hydroxyde, phosphate, hydrogénophosphate, dihydrogénophosphate, chlorure, fluorure, nitrate, benzoate, oxalate, sans que ces citations soient limitatives.

Le rapport molaire p est de préférence compris entre 0,02 et 0,2.

Si l'on se réfère aux techniques générales de préparation de phosphates (telles que décrites notamment dans "PASCAL P. Nouveau traité de chimie minérale" tome X (1956), pages 821-823 et dans "GMELINS Handbuch der anorganischen Chemie" (8^{ème} édition) volume 16 (C), pages 202-206 (1965), on peut distinguer deux voies principales d'accès aux phosphates. D'une part, la précipitation d'un sel soluble du métal (chlorure, nitrate) par l'hydrogénophosphate d'ammonium ou l'acide phosphorique.D'autre part, la dissolution de l'oxyde ou du carbonate du métal (insolubles) avec de l'acide phosphorique, généralement à chaud, suivie d'une reprécipitation.

Les phosphates précipités obtenus selon l'une des voies indiquées peut être séché, traité par une base organique (telle que l'ammoniaque) ou minérale (telle qu'un hydroxyde de métal alcalin) et être soumis à une calcination, ces trois opérations pouvant être réalisées dans l'ordre indiqué ou dans un ordre différent.

Les phosphates métalliques de formule (II) pour lesquels le symbole p est supérieur à 0, peuvent être préparés par imprégnation du composé (PO₄)ₙ Hₙ M préparé selon l'une des techniques décrites précédemment, avec une solution ou une suspension de Imp dans un solvant volatil, tel que l'eau de préférence.

Les résultats sont d'autant meilleurs que Imp est plus soluble et que le composé (PO₄)ₙ Hₕ M est plus fraîchement fabriqué.

Ainsi un procédé avantageux de préparation des phosphates de formule (II) consiste :
a) à réaliser la synthèse du composé (PO₄)ₙ Hₕ M ; puis de préférence sans séparer (PO₄)ₙ Hₕ M du milieu réactionnel ;
b) à introduire l'imprégnant Imp dans le milieu réactionnel ;
c) à séparer l'éventuel liquide résiduel d'avec le solide réactionnel ;
d) à sécher et éventuellement à calciner.

Les performances du catalyseur de formule (II) et notamment sa résistance à la désactivation peuvent être encore améliorées par une calcination. La température de calcination sera avantageusement comprise entre 300°C et 1000°C et de préférence entre 400°C et 900°C. La durée de la calcination peut varier dans de larges limites. A titre indicatif, elle se situe généralement entre 1 heure et 24 heures.

Parmi les catalyseurs de formule (II) préférés dans le procédé de l'invention, on peut citer plus particulièrement le phosphate de lanthane, le phosphate de lanthane calciné, le phosphate de lanthane associé à un dérivé du césium, du rubidium ou du potassium, le phosphate de cérium calciné, le phosphate de cérium associé à un composé du césium, du rubidium ou du potassium, le phosphate de samarium associé à un composé du césium, du rubidium ou du potassium, le phosphate d'aluminium, le phosphate d'aluminium associé à un composé du césium, du rubidium ou du potassium, le phosphate de niobium calciné, le phosphate de niobium associé à un composé du césium, du rubidium ou du potassium, l'hydrogénophosphate de zirconium calciné, l'hydrogénophosphate de zirconium associé à un composé du césium, du rubidium ou du potassium.

Généralement le catalyseur est mis en oeuvre sous forme de poudre, de pastilles, de billes ou d'extrudés, ladite mise en forme pouvant éventuellement être réalisée à l'aide d'un liant. Il peut dans certain cas être avantageux qu'au moins une partie du volume libre du réacteur soit occupé par un solide inerte, tel que par exemple du quartz, afin de favoriser la vaporisation et la dispersion des réactifs.

La réaction d'hydrolyse cyclisante nécessite la présence d'eau. Le rapport molaire entre l'eau et l'aminonitrile engagés se situe habituellement entre 0,5 et 50 et de préférence entre 1 et 20.

L'aminonitrile et l'eau peuvent être engagés sous forme de leurs mélanges à l'état de vapeurs ou être introduits séparément dans le réacteur. On peut réaliser une prévaporisation des réactifs qui circulent ensuite dans une chambre de mélange.

On peut sans inconvénient utiliser tout gaz inerte comme vecteur, tel que l'azote, l'hélium ou l'argon.

La température à laquelle est mis en oeuvre le procédé de l'invention doit être suffisante pour que les réactifs soient bien à l'état de vapeurs. Elle se situe généralement entre 200°C et 450°C et de préférence entre 250°C et 400°C.

Le temps de contact entre l'aminonitrile et le catalyseur n'est pas critique. Il peut varier selon l'appareillage utilisé notamment. Des temps de contact de 1 à 200 secondes sont préférés et encore plus préférentiellement de 50 à 100 secondes.

La pression n'est pas un paramètre critique du procédé. Ainsi on peut opérer sous des pressions de 10⁻³ bar à 200 bars. De préférence on mettra en oeuvre le procédé sous une pression de 0,1 à 20 bars.

Il n'est pas exclu d'utiliser un solvant inerte dans les conditions réactionnelles, tel que par exemple un alcane, un cycloalcane, un hydrocarbure aromatique ou l'un de ces hydrocarbures précédents halogéné, et d'avoir ainsi une phase liquide dans le flux réactionnel.

Les exemples qui suivent illustrent l'invention.

### EXEMPLES 1 A 6

Dans un réacteur cylindrique de 20 ml en verre Pyrex, disposé verticalement et muni de moyens de chauffage, d'ouvertures pour l'arrivée et la sortie des flux gazeux et d'un système d'injection des réactifs, on charge successivement 10 ml de quartz, 1ml du catalyseur sous forme de poudre de 0,8 à 1,25 micromètre (nature du catalyseur indiquée dans le tableau 1 ci-après) et à nouveau 10 ml de quartz.

Le réacteur ainsi chargé est chauffé à 400°C sous courant d'air (avec un débit de 1,5 litre/heure) pendant 2 heures. Ensuite le réacteur est refroidi à 320°C (température de réaction choisie) et mis sous courant d'azote (débit de 1 litre/heure).

On injecte alors, à l'aide d'une pompe, un mélange d'amino-6 capronitrile (ACN) et d'eau (rapport pondéral 50/50, soit un rapport molaire eau/ACN de 6,2). La vitesse d'injection du mélange est de 1,2 ml/h.

A la sortie du réacteur, les vapeurs sont condensées dans un piège en verre à température ambiante, sur une durée de 2 heures.

Le mélange réactionnel final est dosé en chromatographie en phase vapeur.

On détermine le taux de transformation (TT) de l'aminocapronitrile, le rendement (RT) en caprolactame (CPL) par rapport à l'aminocapronitrile transformé et l'activité du catalyseur mesurée en grammes de caprolactame formé/millilitre de catalyseur.heure.

Le tableau 1 ci-après rassemble les résultats obtenus.

**Tableau 1**

| **Exemples** | **Catalyseur** | **TT % ACN** | **RT % CPL** | **Activité** |
|---|---|---|---|---|
| Exemple 1 | LaPO4 | 63 | 91 | 0,49 g |
| Exemple 2 | LaPO4 calciné 4 h à 700°C | 90 | 92 | 0,47 g |
| Exemple 3 | LaPO4/Cs * | 69 | 97 | 0,63 g |
| Exemple 4 | AlPO4 | 88 | 93 | 0,70 g |
| Exemple 5 | Zr(HPO4)2 | 45 | 77 | 0,32 g |
| Exemple 6 | NbOPO4 | 39 | 100 | 0,33 g |

| | | | | |
|---|---|---|---|---|
| * phosphate de La dopé par l'hydrogénophosphate de Cs (rapport molaire Cs/LaPO₄ = 0,053) | | | | |

### EXEMPLES 7 A 9

On répète les exemples 1 à 3, en suivant l'évolution de l'activité des différents catalyseurs sur des durées allant jusqu'à 35 heures.

Le tableau 2 ci-après rassemble les valeurs de l'activité pour chaque catalyseur et pour des durées croissantes de réaction (ND = valeur non déterminée).

**Tableau 2**

| Exemples | Catalyseur | Activité du catalyseur pour des durées de | | | | | | |
|---|---|---|---|---|---|---|---|---|
| | | 4 h | 6 h | 8 h | 10 h | 25 h | 30 h | 35 h |
| Exemple 7 | LaPO4 | 0,45 | 0,40 | 0,35 | ND | ND | ND | ND |
| Exemple 8 | LaPO4/Cs | 0,60 | 0,55 | 0,60 | 0,68 | 0,60 | 0,55 | 0,63 |
| Exemple 9 | LaPO4 calciné | 0,62 | 0,70 | 0,63 | 0,50 | 0,35 | 0,37 | 0,42 |

### EXEMPLES 10 ET 11

On répète l'exemple 1 dans les mêmes conditions, mais en opérant la réaction d'hydrolyse cyclisante à des températures différentes.

Le tableau 3 ci-après rassemble les résultats obtenus et rappelle également les résultats de l'exemple 1 à titre comparatif.

**Tableau 3**

| Exemples | Température | TT % ACN | RT % CPL | Activité |
|---|---|---|---|---|
| Exemple 10 | 280°C | 44 % | 90 % | 0,4 |
| Exemple 11 | 300°C | 46 % | 100 % | 0,5 |
| Exemple 1 | 320°C | 63 % | 91 % | 0,5 |

### ESSAI COMPARATIF A

On répète l'exemple 1, en remplaçant le phosphate de La par de la silice (commercialisée sous la marque Aerosil 200), calcinée pendant 16 h à 600°C.

Les conditions opératoires sont les mêmes que pour l'exemple 1 (température de 320°C ; rapport molaire eau/ACN de 6,2 ; durée de 2 h).

On a obtenu les résultats suivants :
- TT de l'ACN : 17,6 %
- RT en CPL : 53 %
- Activité du catalyseur : 0,04 g CPL/ml catalyseur.h

La silice conduit à un faible rendement en caprolactame et présente une activité extrêmement basse.

## Revendications

1. Procédé de préparation de lactame par réaction en phase vapeur d'un aminonitrile aliphatique de formule générale (I) :
N≡C-R-NH₂ (I)
dans laquelle R représente un radical alkylène ayant de 3 à 12 atomes de carbone, avec de l'eau, en présence d'un catalyseur solide, caractérisé en ce que le catalyseur est un phosphate métallique de formule générale (II) :
(PO₄)ₙ Hₕ M, (Imp)ₚ (II)
dans laquelle :
- M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O,
- Imp représente un composé d'imprégnation basique constitué d'un métal alcalin ou alcalino-terreux, ou de mélanges de plusieurs de ces métaux, associé à un contre-anion pour assurer la neutralité électrique,
- n représente 1, 2 ou 3,
- h représente 0, 1 ou 2,
- p représente un nombre compris entre 0 et 1/3 et correspond à un rapport molaire entre l'imprégnant Imp et l'imprégné (PO₄)ₙ Hₕ M.

2. Procédé selon la revendication 1, caractérisé en ce que l'aminonitrile de formule (I) est l'amino-6 capronitrile.

3. Procédé selon l'une des revendications 1 ou 2, caractérisé en ce que dans la formule (II) des phosphates métalliques, les métaux des groupes 2a, 3b, 4b ,5b, 6b, 7b, 8, 2b, 3a, 4a et 5a de la classification périodique des éléments représentés par M sont choisis parmi le béryllium, le magnésium, le calcium, le strontium, le baryum, l'aluminium, le bore, le gallium, l'indium, l'yttrium, les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium, le zirconium, le titane, le vanadium, le niobium, le fer, le germanium, l'étain, le bismuth.

4. Procédé selon l'une des revendications 1 à 3, caractérisé en ce que l'on met en oeuvre des phosphates métalliques de formule (II) qui sont des mélanges de phosphates de plusieurs des métaux M ou des phosphates mixtes de plusieurs des métaux M ou encore des phosphates mixtes contenant un ou plusieurs des métaux M et un ou plusieurs autres métaux tels que les métaux alcalins ou alcalino-terreux.

5. Procédé selon l'une des revendications 1 à 4, caractérisé en ce que les contre-anions entrant dans la formule du composé d'imprégnation Imp sont basiques et sont choisis de préférence parmi les ions hydroxyde, phosphate, hydrogénophosphate, dihydrogénophosphate, chlorure, fluorure, nitrate, benzoate, oxalate.

6. Procédé selon l'une des revendications 1 à 5, caractérisé en ce que le rapport molaire p est de préférence compris entre 0,02 et 0,2.

7. Procédé selon l'une des revendications 1 à 6, caractérisé en ce que le catalyseur de formule (II) est soumis à une calcination à une température comprise entre 300°C et 1000°C et de préférence entre 400°C et 900°C.

8. Procédé selon l'une des revendications 1 à 7, caractérisé en ce que le catalyseur est plus particulièrement choisi parmi le phosphate de lanthane, le phosphate de lanthane calciné, le phosphate de lanthane associé à un dérivé du césium, du rubidium ou du potassium, le phosphate de cérium calciné, le phosphate de cérium associé à un composé du césium, du rubidium ou du potassium, le phosphate de samarium associé à un composé du césium, du rubidium ou du potassium, le phosphate d'aluminium, le phosphate d'aluminium associé à un composé du césium, du rubidium ou du potassium, le phosphate de niobium calciné, le phosphate de niobium associé à un composé du césium, du rubidium ou du potassium, l'hydrogénophosphate de zirconium calciné, l'hydrogénophosphate de zirconium associé à un composé du césium, du rubidium ou du potassium.

9. Procédé selon l'une des revendications 1 à 8, caractérisé en ce que le rapport molaire entre l'eau et l'aminonitrile engagés se situe entre 0,5 et 50 et de préférence entre 1 et 20.

10. Procédé selon l'une des revendications 1 à 9, caractérisé en ce que la température à laquelle est mis en oeuvre le procédé de l'invention se situe entre 200°C et 450°C et de préférence entre 250°C et 400°C.

## Patentansprüche

1. Verfahren zur Herstellung von Laktam durch Reaktion in der Gasphase eines aliphatischen Aminonitrils der allgemeinen Formel (I):
N≡C-R-NH₂ (I)
in der R einen Alkylenrest mit 3 bis 12 Kohlenstoffatomen darstellt, mit Wasser in Gegenwart eines festen Katalysators, dadurch gekennzeichnet, dass der Katalysator ein Metallphosphat der allgemeinen Formel (II):
(PO₄)ₙHₕM, (Imp)ₚ (II)
ist, in der:
- M ein zweiwertiges, dreiwertiges, vierwertiges oder fünfwertiges Element, das unter den Gruppen 2a, 3b, 4b, 5b, 6b, 7b, 8, 2b, 3a, 4a und 5a des Periodensystems der Elemente ausgewählt ist, oder ein Gemisch von mehreren dieser Elemente darstellt, oder M = O,
- Imp eine basische Tränkverbindung darstellt, die aus einem Alkali- oder Erdalkalimetall oder aus Gemischen von mehreren dieser Metalle besteht, gebunden an ein Gegenion, um die elektrische Neutralität zu gewährleisten,
- n 1, 2 oder 3 darstellt,
- h 0, 1 oder 2 darstellt,
- p eine Zahl zwischen 0 und 1/3 darstellt und einem Molverhältnis zwischen dem Tränkmittel Imp und der getränkten Masse (PO₄)ₙHₕM entspricht.

2. Verfahren gemäß Anspruch 1, dadurch gekennzeichnet, dass das Aminonitril der Formel (I) 6-Aminocapronitril ist.

3. Verfahren gemäß einem der Ansprüche 1 oder 2, dadurch gekennzeichnet, dass in der Formel (II) der Metallphosphate die durch M dargestellten Metalle der Gruppen 2a, 3b, 4b, 5b, 6b, 7b, 8, 2b, 3a, 4a und 5a des Periodensystems der Elemente unter Beryllium, Magnesium, Calcium, Strontium, Barium, Aluminium, Bor, Gallium, Indium, Yttrium, den Lanthaniden, wie Lanthan, Cer, Praseodym, Neodym, Samarium, Europium, Gadolinium, Terbium, Dysprosium, Holmium, Erbium, Thulium, Ytterbium und Lutetium, Zirkonium, Titan, Vanadium, Niob, Eisen, Germanium, Zinn, Wismuth ausgewählt sind.

4. Verfahren gemäß einem der Ansprüche 1 bis 3, dadurch gekennzeichnet, dass man Metallphosphate der Formel (II) einsetzt, die Gemische von Phosphaten von mehreren der Metalle M oder Mischphosphate von mehreren der Metalle M oder auch Mischphosphate, die eines oder mehrere der Metalle M und ein oder mehrere weitere Metalle, wie die Alkali- oder Erdalkalimetalle, enthalten, sind.

5. Verfahren gemäß einem der Ansprüche 1 bis 4, dadurch gekennzeichnet, dass die Gegenionen, die in die Formel der Tränkverbindung Imp eingehen, basisch sind und vorzugsweise unter den Hydroxid-, Phosphat-, Hydrogenphosphat-, Dihydrogenphosphat-, Chlorid-, Fluorid-, Nitrat-, Benzoat-, Oxalationen ausgewählt sind.

6. Verfahren gemäß einem der Ansprüche 1 bis 5, dadurch gekennzeichnet, dass das Molverhältnis p vorzugsweise zwischen 0,02 und 0,2 liegt.

7. Verfahren gemäß einem der Ansprüche 1 bis 6, dadurch gekennzeichnet, dass der Katalysator der Formel (II) einem Glühen bei einer Temperatur zwischen 300 °C und 1000 °C und vorzugsweise zwischen 400 °C und 900 °C unterzogen wird.

8. Verfahren gemäß einem der Ansprüche 1 bis 7, dadurch gekennzeichnet, dass der Katalysator spezieller unter Lanthanphosphat, geglühtem Lanthanphosphat, an ein Cäsium-, Rubidium- oder Kaliumderivat assoziiertem Lanthanphosphat, geglühtem Cerphosphat, an eine Cäsium- Rubidium- oder Kaliumverbindung assoziiertem Cerphosphat, an eine Cäsium-, Rubidium- oder Kaliumverbindung assoziiertem Samariumphosphat, Aluminiumphosphat, an eine Cäsium-, Rubidium- oder Kaliumverbindung assoziiertem Aluminiumphosphat, geglühtem Niobphosphat, an eine Cäsium-, Rubidium- oder Kaliumverbindung assoziiertem Niobphosphat, geglühtem Zirkoniumhydrogenphosphat, an eine Cäsium-, Rubidium- oder Kaliumverbindung assoziiertem Zirkoniumhydrogenphosphat ausgewählt ist.

9. Verfahren gemäß einem der Ansprüche 1 bis 8, dadurch gekennzeichnet, dass das Molverhältnis zwischen dem Wasser und dem Aminonitril, die eingesetzt werden, zwischen 0,5 und 50 und vorzugsweise zwischen 1 und 20 liegt.

10. Verfahren gemäß einem der Ansprüche 1 bis 9, dadurch gekennzeichnet, dass die Temperatur, bei der das Verfahren der Erfindung durchgeführt wird, zwischen 200 °C und 450 °C und vorzugsweise zwischen 250 °C und 400 °C liegt.

## Claims

1. Process for preparing a lactam by the vapour-phase reaction of aliphatic aminonitrile of general formula (I):
N≡C-R-NH₂ (I)
in which R represents an alkylene radical containing from 3 to 12 carbon atoms, with water, in the presence of a solid catalyst, characterized in that the catalyst is a metal phosphate of general formula (II):
(PO₄)ₙHₕM, (Imp)ₚ (II)
in which:
- M represents a divalent, trivalent, tetravalent or pentavalent element chosen from groups 2a, 3b, 4b, 5b, 6b, 7b, 8, 2b, 3a, 4a and 5a of the Periodic Table of the Elements, or a mixture of several of these elements, or M = O,
- Imp represents a basic impregnating compound consisting of an alkali metal or alkaline-earth metal, or mixtures of several of these metals, combined with a counteranion to ensure the electrical neutrality,
- n represents 1, 2 or 3,
- h represents 0, 1 or 2,
- p represents a number between 0 and 1/3 and corresponds to a molar ratio between the impregnating compound Imp and the impregnated compound (PO4)ₙHₕM.

2. Process according to claim 1, characterized in that the aminonitrile of formula (I) is 6-amino capronitrile.

3. Process according to either of claims 1 and 2, characterized in that, in formula (II) of the metal phosphates, the metals from groups 2a, 3b, 4b, 5b, 6b, 7b, 8, 2b, 3a, 4a and 5a of the Periodic Table of the Elements represented by M are chosen from beryllium, magnesium, calcium, strontium, barium, aluminium, boron, gallium, indium, yttrium, lanthanides such as lanthanum, cerium, praseodymium, neodymium, samarium, europium, gadolinium, terbium, dysprosium, holmium, erbium, thulium, ytterbium, lutetium, zirconium, titanium, vanadium, niobium, iron, germanium, tin and bismuth.

4. Process according to one of claims 1 to 3, characterized in that metal phosphates of formula (II) are used which are mixtures of phosphates of several of the metals M or mixed phosphates of several of the metals M or alternatively mixed phosphates containing one or more of the metals M and one or more other metals such as alkali metals or alkaline earth metals.

5. Process according to one of claims 1 to 4, characterized in that the counteranions forming part of the formula of the impregnating compound Imp are basic and are preferably chosen from hydroxide, phosphate, hydrogenophosphate, dihydrogenophosphate, chloride, fluoride, nitrate, benzoate and oxalate ions.

6. Process according to one of claims 1 to 5, characterized in that the molar ratio p is preferably between 0.02 and 0.2.

7. Process according to one of claims 1 to 6, characterized in that the catalyst of formula (II) is subjected to a calcination at a temperature between 300°C and 1000°C and preferably between 400°C and 900°C.

8. Process according to one of claims 1 to 7, characterized in that the catalyst is chosen more particularly from lanthanum phosphate, calcinated lanthanum phosphate, lanthanum phosphate combined with a caesium, rubidium or potassium derivative, calcinated cerium phosphate, cerium phosphate combined with a caesium, rubidium or potassium compound, samarium phosphate combined with a caesium, rubidium or potassium compound, aluminium phosphate, aluminium phosphate combined with a caesium, rubidium or potassium compound, calcinated niobium phosphate, niobium phosphate combined with a caesium, rubidium or potassium compound, calcinated zirconium hydrogenophosphate and zirconium hydrogenophosphate combined with a caesium, rubidium or potassium compound.

9. Process according to one of claims 1 to 8, characterized in that the molar ratio between the water and the aminonitrile used is between 0.5 and 50 and preferably between 1 and 20.

10. Process according to one of claims 1 to 9, characterized in that the temperature at which the process of the invention is carried out is between 200°C and 450°C and preferably between 250°C and 400°C.
